(19) Europäisches Patentamt
European Patent Office
Office européen des brevets

(11) EP 4 592 396 A1

(12) EUROPEAN PATENT APPLICATION
published in accordance with Art. 153(4) EPC

(43) Date of publication:
**30.07.2025 Bulletin 2025/31**

(21) Application number: **23867681.1**

(22) Date of filing: **18.09.2023**

(51) International Patent Classification (IPC):
***C12P 19/04*** (2006.01) ***C12P 19/14*** (2006.01)
***C12N 9/24*** (2006.01) ***C07H 3/06*** (2006.01)
***C08B 37/14*** (2006.01) ***C08H 8/00*** (2010.01)

(52) Cooperative Patent Classification (CPC):
**C07H 3/06; C08B 37/14; C08H 8/00; C12N 9/24; C12P 19/04; C12P 19/14**

(86) International application number:
**PCT/ES2023/070562**

(87) International publication number:
**WO 2024/062148 (28.03.2024 Gazette 2024/13)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **19.09.2022 ES 202200077**

(71) Applicant: **Universidade de Vigo**
**36310 Vigo, Pontevedra (ES)**

(72) Inventors:
- **RÚA RODRÍGUEZ, María Luisa**
  **32004 Ourense (ES)**
- **TORRADO AGRASAR, Ana María**
  **32004 Ourense (ES)**
- **FUCIÑOS GONZÁLEZ, Clara**
  **32004 Ourense (ES)**
- **RODRÍGUEZ SANZ, Andrea**
  **32004 Ourense (ES)**

(74) Representative: **Clarke, Modet y Cía., S.L.**
**C/ Suero de Quiñones 34-36**
**28002 Madrid (ES)**

# (54) PROCESS FOR PRODUCING HEMICELLULOSE-DERIVED PRODUCTS

(57) The present invention relates to a process for producing hemicellulose-derived products from a lignocellulosic material comprising an alkaline pretreatment of said lignocellulosic material to generate a hydrolysable solid, and an enzymatic hydrolysis of the solid resulting from step a) for the extraction of the hemicellulose and production of hemicellulose-derived products, where the enzymatic hydrolysis of resulting solid is carried out with an endo-xylanase at a temperature of 55-80 °C.

EP 4 592 396 A1

## Description

FIELD OF THE INVENTION

The present invention falls within the field of study and research of the production of hemicellulose derivatives from lignocellulosic materials.

STATE OF THE ART

Plant biomass is composed of three fractions: cellulose, hemicellulose and lignin. Due to the presence of these compounds, the term lignocellulosic is also used to define those materials that comprise these fractions. Each of these fractions can be used individually to obtain a variety of products.

The wide availability of these materials which, in many cases, are by-products resulting from agricultural and forestry productive activities, their consideration as renewable raw materials, and the diversity of derivatives that can be obtained from each of the aforementioned fractions has led to a growing interest in the extraction of these fractions and their transformation into other compounds in recent years.

Specifically, hemicellulose is a heterogeneous set of polysaccharides other than cellulose. As we have mentioned, these compounds are naturally occurring in cell walls of most plants, along with cellulose. Unlike cellulose, made up of glucose, hemicellulose is made up of different monosaccharides, such as xylose, arabinose, galactose, mannose, glucose and carboxylic acids such as glucuronic, galacturonic and acetic acid.

In the particular case of agricultural residues from the cultivation and processing of cereals, the hemicellulosic fraction is mainly composed of arabinoxylan (xylose polymer with a variable number of arabinose branches), which has a chemical structure according to Formula I.

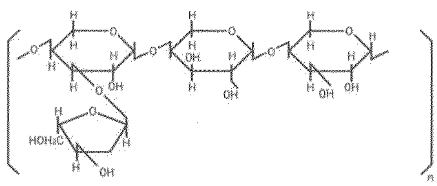

Formula I

Arabinoxylan is of great industrial interest, since from it, it is possible to obtain functional food compounds of high commercial and technological interest such as xylooligosaccharides (XOS) or arabinoxylooligosaccharides (AXOS) with prebiotic properties, or polymers derived from arabinoxylan (ADPs) with properties as texturizing agents, among others.

Xylooligosaccharides (XOS) are understood to be that set of compounds formed by a linear chain of a variable number of D-xylopyranose monomers (between 2 and 10), linked by glycosidic bonds β-(1,4). Xylooligosaccharides can have diverse structures due to the presence of variable branches, dependent on the source material and the treatment of obtaining xylooligosaccharides, consisting of L-arabinofuranosyl, D-glucuronopyranosyl, 4-O-methyl D-glucuronopyranosyl and acetyl units. Arabinoxylooligosaccharides (AXOS) are those XOS that contain one or more arabinose branches. The main interest of XOS and AXOS lies in their beneficial effects on health, validated by different *in vitro* and in *vivo* assays, and in their usefulness as a prebiotic functional ingredient. As mentioned in Rios-Rios, K. L., Dejonghe, W., Vanbroekhoven, K., Rakotoarivonina, H., & Rémond, C. (2021). Enzymatic Production of Xylo-oligosaccharides from Destarched Wheat Bran and the Impact of Their Degree of Polymerization and Substituents on Their Utilization as a Carbon Source by Probiotic Bacteria. Journal of Agricultural and Food Chemistry, 69(44), 13217-13226., the prebiotic effect is greater as the degree of polymerization of XOS decreases. In this sense, in this invention, those oligosaccharides with a degree of polymerization between 2 and 6 (X2-X6) are considered XOS-AXOS.

On the other hand, polymers derived from arabinoxylan (ADPs) are that set of compounds chemically similar to xylooligosaccharides, which have more than 6 xylose units, which have low prebiotic activity.

The three fractions present in plant biomass (cellulose, hemicellulose and lignin) usually form a very compact structure so, for their use individually, they must be treated to reduce the existing interaction between these fractions.

Currently, there are different treatment processes for the fractionation, extraction of hemicellulose and production of its derivatives (XOS and ADPs).

Generally, the most efficient and most used processes for fractionation are those that use high temperatures, high pressures or organic compounds.

Examples of such processes are hydrothermal pretreatments, steam explosion processes, acid pretreatments, as described, for example, in the following patents:

- WO2015107413A1, which describes a continuous chemical-enzymatic sequential process for the fractionation of holocellulose into arabinoxylooligosaccharides, xylooligosaccharides and cellooligosaccharides from agricultural waste;
- ES2364307A1, which describes a process for obtaining substituted xylooligosaccharides from wheat bran, consisting of treating this raw material with water at a temperature high enough to allow the extraction of at least part of the hemicelluloses;
- EP3299394A1, which describes a method for producing a modified xylooligosaccharide with a hydrothermal treatment under high pressure and a temperature of 160°C or higher, followed by an enzymatic hydrolysis; and
- CN1680415A, where the production of xylooligosaccharides from the mixture of powdered corn kernels and water is described, using an acid treatment at high temperature followed by an enzymatic hydrolysis of the extracted soluble fraction.

These processes can extract up to 98% of the hemicellulose in the lignocellulosic material, so the total yield of these processes is high.

However, the conditions required by these treatments make these processes considered environmentally unsustainable. In addition, despite the good yields in the extraction of hemicellulose, these procedures usually involve the generation of unwanted compounds that act as contaminants of the final products obtained. For the use of hemicellulosic fraction derivatives as functional food ingredients, the impurities in the final product are especially undesirable due to their toxic character (some of them such as furfural and 5-hydroxymethylfurfural), and must be eliminated by incorporating additional complex and expensive purification steps.

In this sense, there are numerous limitations on the use of these compounds as food additives. By way of example, Regulation (EU) No 231/2012 sets the limit of furfural in additives, and specifically in guar gum, at a maximum of 1 mg/kg. Toxic effects of 5-Hydroxymethylfurfural (HMF) have also been reported at doses greater than 75 mg/kg body weight (Ulbricht, R.J.; Northup, S.J.; Thomas, J.A. A Review of 5-Hydroxymethylfurfural (HMF) in ParenteralSolutions. Toxicol. Sci. 1984, 4, 843-853).

The generation of xylose is also an undesired effect during the production of XOS because it is not considered a prebiotic compound. Accordingly, Commission Regulation 2018/1648, which authorises the marketing of xylooligosaccharides as a Novel Food, defines maximum xylose levels of 1, 5 or 15% based on the ingredient quality.

Therefore, these fractionation methodologies are not suitable for this type of application, where the degree of impurities present in the final product is relevant.

Alternatively, milder methodologies have been developed that obtain lower yields of hemicellulose extraction. These processes are characterized by a lower severity factor, which can be calculated as follows:

$$(1)\ SF = \log\left(t\ x\ exp^{\left(\frac{(T-100)}{14.75}\right)}\right)$$

where *t* is the incubation time (in minutes) and *T* is the temperature (in °C).

Thus, a mild process can be defined as one carried out at temperatures equal to or lower than 100°C and which has a severity factor lower than SF < 2. Comparably, processes that use a pretreatment with high temperature have an SF > 2.7.

According to these methodologies, an extraction of less than 50% of the hemicellulose from the material is achieved. However, the better quality of the products (in terms of purity due to the lower percentage content of degradation products, such as furfural and 5-hydroxymethylfurfural, and monosaccharides, such as xylose) makes these processes an interesting alternative to those commonly used for the production of hemicellulose derivatives.

In these cases, as in the most intense treatments, a post-extraction step of the hemicellulose can be added for the reduction of the polymer size (herein referred to as ADPs - arabinoxylan *derived polymers*) to XOS with prebiotic effect.

Proof of this is the patent WO2007128025A1, which describes the production of XOS through a hydrothermal treatment or through an enzymatic treatment from a xylan extracted by cold alkaline hydrolysis (CAE). According to this second option, an XOS production of 780 mg XOS per gram of xylan extracted through the enzymatic treatment was achieved, albeit with low xylan extraction yields from the starting material.

Another example of this type of process can be found in the article Chapla, D., Pandit, P., & Shah, A. (2012). Production of xylooligosaccharides from corncob xylan by fungal xylanase and their utilization by probiotics. Bioresource Technology, 115, 215-221. Described herein is the selective production process of xylooligosaccharides (XOS), using partially purified xylanase from *Aspergilus foetidus* MTCC 4898, after alkaline extraction pretreatment. According to this document, the optimal temperature for the production of these xylooligosaccharides was 45°C, since an increase in temperature meant a reduction in the activity of enzyme, which translates into an important limitation of the process.

Alternatively, there are methods based on the application of enzymes on the solid at moderate temperatures,

although these processes have a low yield compared to the first ones. De echo, Yegin, S. (2022). Microbial xylanases in xylooligosaccharide production from lignocellulosic feedstocks. Biomass Conversion and Biorefinery*),* affirm that direct enzymatic hydrolysis on agro-industrial waste is still in a development phase.

In this line is, however, the process described in the article Rodriguez-Sanz, *A.*, *Fuciños, C., Torrado, A. M., & Rúa, M. L. (2022). Extraction of the wheat straw hemicellulose fraction assisted by commercial endo-xylanases. Role of the accessory enzyme activities. Industrial Crops and Products, 179,* 114655. This article describes a process comprising a cold alkaline extraction (CAE) pretreatment. In this sense, the method comprises a NaOH pretreatment (10 and 100 g/L) for 90 minutes at 40°C and 340 rpm in an orbital incubator, with a 1:14 wheat straw:liquid ratio. Subsequently, a neutralization with 37% HCl with a pH of 5.5 - 7.0 of the solid part resulting from the NaOH treatment and a wash with distilled water were carried out until the conductivity was less than 25 $\mu$S/cm. Finally, the samples were dried in an oven at 70°C. After an initial sterilization of 1h at 100°C, the enzymatic hydrolysis of the pretreated solid part was carried out, using three types of enzymatic cocktails (*Ultraflo L, Shearzyme 500 L,* and *Pentopan® Mono Concentrate*) at a temperature of 40°C and a pH of 5.0 in a reaction mixture consisting of 1 g of the pretreated solid fraction, resuspended in 42 ml of a buffer solution and 200 U of endo-xylanase activity. The best result was achieved with Ultraflo, which allowed the extraction of about 60% of the hemicellulose from the solid, but generated a relevant amount of undesirable xylose and arabinose monomers for the generation of XOS and AXOS for applications such as prebiotics.

By way of summary of all the above, it can be said that the different solutions described so far for the extraction of hemicellulose and production of its derivatives (XOS and ADPs) present multiple drawbacks.

Firstly, there are solutions that use a hydrothermal treatment, with a high severity factor due to the high temperature required in this type of treatment, so they are unsustainable processes with the environment. In addition, these processes generate a greater presence of impurities in the resulting products, which limits their use in some sectors, such as food. Alkaline extraction methods are also used at moderate temperatures for hemicellulose solubilization without degradation compounds, which generate low extraction yields, as well as processes based on the use of enzymes directly on solid materials, even in incipient phases of development.

Generally, in addition, current enzymatic processes are designed for XOS and AXOS generation as final products, no process based on the use of enzymes that allows to produce polymers derived from arabinoxylan (ADPs) and/or low molecular weight xylooligosaccharides (XOS and AXOS) in a directed manner has been described.

In order to jointly overcome the limitations reported in the state of the art with respect to the extraction yields of hemicellulose and toxic compounds generation, and allow the targeted production of two differentiated hemicellulose-derived products (XOS-AXOS and ADPs), a new process for the production of hemicellulose derivatives is proposed.

## DESCRIPTION OF THE INVENTION

The present invention describes a new process for producing hemicellulose-derived products.

It is a sustainable and efficient process, with low energy demand, based on the use of enzymes, which allows generation of products derived from hemicellulose to be directed towards obtaining low molecular weight compounds (XOS-AXOS) or medium-high molecular weight compounds (ADPs) depending on the temperature of the process, also improving the extraction yields of hemicellulose obtained by current gentle treatments.

The process comprises two steps:

a) an alkaline pretreatment of the lignocellulosic material to generate a hydrolysable solid, and

b) an enzymatic hydrolysis of the solid resulting from step a) for the extraction of the hemicellulose and the targeted production of low (XOS-AXOS) and medium-high molecular weight (ADPs) hemicellulose-derived products.

Due to the process described in the present invention, the targeted production of xylooligosaccharides and arabinoxylooligosaccharides (XOS and AXOS) and arabinoxylan-derived polymers (ADPs), from lignocellulosic materials, (preferably from cereals due to their composition), is achieved in a sustainable and efficient manner, so its application can be of great interest to the sector dedicated to the use of these lignocellulosic materials and to the production of these compounds.

The alkaline pretreatment at low temperature proposed in step a) of the present invention is carried out to obtain a solid hydrolyzable by endo-xylanases, avoiding the hemicellulose fraction extraction and the generation of toxic carbohydrate-degrading compounds. In contrast to existing hydrothermal processes, step a) of the production process of hemicellulose-derived products according to the present invention has a severity factor lower than 2, preferably in the range between -0.5 and 2. In this sense, the alkaline treatment, for example, using 10g/L NaOH, for 90 min at 40°C has a SF factor = 0.2. By including the autoclave treatment prior to enzymatic hydrolysis, the maximum severity factor achieved throughout the process can reach a value around SF = 1.7.

In a preferred embodiment of the pretreatment, the alkaline pretreatment may be initiated with a preconditioning step of solid material by drying at moderate temperatures (preferably below 70°C) to reduce the moisture to values in the

range 3 - 10%, and/or by grinding or milling to reduce the particle size to a lower length or diameter, preferably between 0.5 - 5 cm.

Next, an alkaline treatment of the lignocellulosic solid material is carried out at moderate temperature designed to reduce the lignin content, break the cross-links between xylan polymers due to differentiated bridges and remove the acetyl residues of the arabinoxylan. To this end, said lignocellulosic material is treated with 2-20 g/L of caustic soda (NaOH) for a time not exceeding 90 min and at a temperature in the range 30 - 50°C, preferably in 35 - 45°C, and more preferably at 40°C, in the proportion of 30 - 75 g of straw/L of NaOH.

The solid resulting from this treatment preferably maintains between 70-100% of initial hemicellulose content. This solid is subjected to a pH adjustment treatment, preferably in the range 4-10, which is selected based on the optimal pH of the enzyme to be employed in step b) of enzymatic hydrolysis according to the present invention. For this, the necessary amount of a concentrated solution of food-grade acid, such as HCl, is added.

Once the desired pH is reached, the supernatant is removed by filtration or centrifugation and the pretreated solid is washed with water to remove excess salts generated as a result of the pH adjustment step. Additionally, the resulting wet solid can be subjected to a drying process, for example, in an oven with aeration at a temperature between 60 - 70°C for storage and subsequent use. This solid fraction will be destined for the enzymatic hydrolysis of step b) of the present invention.

Finally, a heat treatment at a temperature not exceeding 100°C for 1h is applied to the pretreated solid in order to ensure maximum hydration of material and open the lignocellulosic matrix in order to facilitate the access of the enzymes to the innermost areas of solid particles. In addition, this heat treatment allows the material to be sterilized and reduces the possibility of subsequent material contamination.

To carry out this heat treatment the pretreated solid is resuspended in the selected liquid medium (water or buffer) to carry out the enzymatic hydrolysis in a 1:40-1:100 solid:liquid ratio.

Once the lignocellulosic solid raw material has been pretreated, step b) of the process proposed in the present invention for the targeted production of hemicellulose-derived products is the enzymatic hydrolysis of said resulting solid fraction.

Regarding the enzymatic hydrolysis treatment, the state of the art establishes that 45°C is the maximum and optimal temperature to carry out enzymatic hydrolysis on soluble hemicelluloses when mesophilic xylanases are used, since from that temperature enzymatic inactivation occurs due to the increase in temperature.

In contrast, the present invention provides that the enzymatic hydrolysis is carried out on the solid part of raw material pretreated at temperatures between 55-80°C.

The result of this enzymatic hydrolysis is the generation of a liquid supernatant composed of a mixture of variable composition of: low molecular weight xylooligosaccharides and arabinoxylooligosaccharides (XOS and AXOS) with polymerization degree between 2 and 6 xylose units with prebiotic effect, high molecular weight arabinoxylan-derived polymers (ADPs) with polymerization degree greater than 6 with technological properties, and a remaining solid rich in cellulose.

By working in these conditions, it is possible to avoid the thermal carbohydrate degradation compounds formation (furfural and HMF), or reduce their generation to concentrations below 5 mg/L, which act as contaminants of final product, especially in products intended for the food sector. Therefore, as they do not have this contaminants type, the hemicellulose-derived products obtained by the process described in the present invention have characteristics suitable for use as food ingredients.

As is known from the state of the art, enzymatic hydrolysis in temperature ranges of 35-45°C (preferably at 40°C) on the pretreated solid allows obtaining a liquid supernatant composed of XOS and AXOS and a low proportion of monomers.

However, the increase in the hydrolysis temperature from ranges from 35-45°C to 55-80°C allows the molecular weight profile of hemicellulose-derived products to be modified, giving rise to arabinoxylan-derived polymers (ADPs), in addition to XOS-AXOS and a low proportion of monomers. Thus, the present invention preferably establishes a hydrolysis at higher temperatures for the simultaneous obtaining of XOS-AXOS and ADPs, using thermostable thermophilic or mesophilic endo-xylanases. Thus, in a preferred embodiment for obtaining these products, the enzymatic hydrolysis is carried out with 200-500 U/g solid pretreated with mesophilic enzymes for 2-50 h.

In addition to this temperature-modulating effect of enzymatic hydrolysis step on the molecular weight profile of final products (not described so far), this increase in the temperature of the enzymatic step offers two advantages.

On the one hand, the increase in temperature favors the activity of active endo-xylanases in a wide range of temperatures, that is, the activity is higher at a higher temperature. In other words, considering two hydrolysis processes, one at 40°C and the other at 65°C with the same enzyme units, a lower contribution of enzymes, by weight, is required in the process at 65°C than those necessary in the process at 40°C to achieve the same enzymatic activity. Likewise, increasing enzyme activity with temperature significantly reduces operating times, which is an important advantage in industrial processes.

On the other hand, the increase in temperature of enzymatic hydrolysis to a range between 55-80°C, implies

greater access to the hemicellulose present in the lignocellulosic material to be treated. That is, the use of a high temperature as described in the present invention generates a greater opening in the lignocellulose structure and the endo-xylanases can better access the xylan bonds resulting in a higher hemicellulose extraction yield with respect to the results described in the state of the art.

Therefore, the effect obtained by increasing the temperature applied during the enzymatic treatment with endo-xylanases on the characteristics of the final products with high extraction yields is a new concept for the processes for obtaining hemicellulose-derived products, such as XOS, AXOS and/or ADPs.

Obtaining XOS-AXOS at low temperature and appearance of ADPs at high temperature can be seen from the beginning of the reaction, although the low yield of the extraction in the early stages of the process may mean less interest in reducing the operation time.

The modulating effect of molecular weight profile of hemicellulose-derived products occurs from the start of the reaction regardless of the hydrolysis time, so that in the temperature range of 35-45°C the generation of ADPs is not detected at any time, while in the temperature range of 55-80°C ADPs are detected at all times, including long reaction times, although the low yield of the extraction in the early stages of the process may mean less interest in reducing the operation time.

In a preferred embodiment of the present invention for the production of XOS-AXOS mixtures and ADPs, the enzymatic step is carried out with thermophilic enzymes at 55-80°C, and in an alternative embodiment and is carried out with thermostable mesophilic enzymes within the range 55-65°C. For the alternative embodiment, the thermostable mesophilic enzymes will be added successively every 2-5 h of hydrolysis, with a total number of 2-10 additions to alleviate the effects of loss of high temperature activity.

Mesophilic enzymes are those that have optimal activity at temperatures in the range 25-45°C, while thermophilic enzymes are those that work at temperatures higher than 45°C and usually have optimal activity at temperatures between 60-80°C. In this sense, it is noteworthy that, in some cases, mesophilic enzymes are also acceptable for carrying out the process according to the present invention at temperatures in the range 55-65°C because they have sufficient partial thermostability to allow them to be active for a certain time interval.

Initially, it can be considered that a greater amount of enzyme accelerates the process of obtaining hemicellulose derivatives. However, it should also be taken into account that a greater amount of enzyme at the same time of hydrolysis produces a greater amount of monomers, which may be undesirable for subsequent uses. Thus, in a preferred embodiment of the present invention, an enzyme dose is set in a range of 200-500 U/g of pretreated solid, the units of enzyme activity being measured at the temperature employed in the process. The selection of the enzymatic working units involves hydrolysis time variation, being important variables to be controlled to avoid the exhaustive generation of xylose (contaminants),

Thus, in a preferred embodiment of the present invention, the enzymatic hydrolysis is carried out for a time of 2 to 50 hours. The hydrolysis time is influenced by the added enzymatic units, with the more enzymatic units, the less total hydrolysis time. The control of these variables is necessary to avoid the generation of monomers at long reaction times.

Regardless of the enzyme and doses used, and in parallel to the effect of temperature on the profile of majority products generated in each case, the increase in temperature generally causes a significant increase in the overall xylan extraction yield. This translates into lower overall process yields when it is directed towards the majority production of XOS and AXOS since in these conditions low temperatures between 35-45°C are applied.

To achieve both ends: high overall xylan utilization yields and high percentages of XOS and AXOS in the final product, in an alternative embodiment, the process according to the present invention comprises a two-step enzymatic hydrolysis.

Thus, in a first step (B1), the enzymatic hydrolysis is carried out at a temperature between 55-80°C with thermophilic endo-xylanases or a thermostable mesophilic endo-xylanase with multiple additions with the aim of extracting as much hemicellulose as possible in the form of ADPs. In the alternative embodiment using a thermostable mesophilic endo-xylanase, it is preferable to use a temperature between 55 and 65°C.

The second step (B2) of the enzymatic hydrolysis is carried out at a lower temperature, between 35-45°C, and preferably around 40°C, using mesophilic endo-xylanases that will preferably act on the ADPs previously generated to finally produce XOS and AXOS as majority final products.

In an even more preferred embodiment, independently, each of the steps, B1 and/or B2, can be carried out with enzyme doses of 200-500 U/g pretreated solid and for times of 2-50 h.

In an even more preferred embodiment, the dose of enzyme will be 200-500 U/g of pretreated solid with successive additions every 2-5 h of hydrolysis with a total number of 2-10 additions. This alternative is especially relevant for a process where a thermostable mesophilic endo-xylanase is used at a temperature between 55-65°C.

In brief, unlike the solutions described in the state of the art based on enzymatic treatments on soluble hemicellulose to obtain xylan derivatives, which employ a single moderate temperature that leads to the generation of xylooligosaccharides and arabinoxylooligosaccharides (XOS and AXOS), either with low overall extraction yields of the hemicellulose in the absence of impurities or with high yields in the presence of impurities, the use of a process as

described in the present invention, which includes an enzymatic hydrolysis step on the pretreated solid in the range 55-80°C as a step after an alkaline pretreatment at low temperature, manages to direct the production of hemicellulose-derived products towards the generation of ADPs simultaneously with XOS and AXOS, or XOS and AXOS in the absence of ADP, with high extraction yields and without generation of undesirable co-products, which is an advantage, for example, for preparation of ingredients for food industry with different applications as prebiotics in the case of XOS and AXOS, or as ingredients with textural properties in the case of ADPs.

These food ingredients (XOS-AXOS and ADPs) present in the liquid fraction will finally be separated from the solid fraction prior to its application according to step c) of the present invention. Thus, in a preferred embodiment, the enzymatic inactivation will be carried out with pH modification of liquid fraction to values far from the optimums of activity and stability of enzyme, followed by a physical filtration treatment or centrifugation for removal of solid residue.

BRIEF DESCRIPTION OF THE DRAWINGS

Figure 1 shows a comparative graph of xylose and arabinose monomers generation (Monom.) from wheat straw hemicellulose, expressed in mg/g of pretreated material, and their percentage with respect to the total hemicellulose extracted, for each of the operating conditions listed in Table 1.

DETAILED DESCRIPTION OF THE INVENTION

In order to describe the present invention, the following example is presented for the production of hemicellulose-derived products from a lignocellulosic material, preferably from cereals.

Specifically, wheat straw of varying sizes (less than 3 cm in length) was employed as the lignocellulosic material. Thus, 300 g of straw were pretreated with 10 g/L caustic soda in a 1:14 liquid solid ratio in a 5 L bioreactor. The treatment was carried out for 90 min at 40°C with stirring and aeration to keep the straw in suspension.

Next, the resulting solid part was neutralized with 37% HCl to a pH of 6.8, and washed until the conductivity was less than 25 μS/cm. Specifically, a conductivity of 22 μS/cm was resulted. Finally, drying was carried out in an oven at 70°C overnight.

After the solid fraction was washed, a heat treatment was applied at a temperature of 100°C for one hour while it was suspended in 25 mM citrate-phosphate buffer pH 5 (for commercial mesophilic enzyme *Pentopan® Mono Concentrate* -PMC-) or pH 6.5 (for the thermophilic enzyme *XynA3),* means selected to carry out the enzymatic hydrolysis in a 1:42 solid:liquid ratio.

From the pretreated solid fraction, 7 enzymatic hydrolysis experiments (E1 - E7) were carried out whose operating conditions were modified: temperature (40°C-65°C), used enzyme (XynA3 and *Pentopan® Mono Concentrate -PMC-*) and enzymatic units (200, 400 and 2000 U/g pretreated solid).

The results of enzymatic hydrolysis can be expressed as a function of extraction yield of hemicellulose (Rto extr.) and the percentage of final products (monomers, XOS-AXOS and ADPs) in the liquid fraction with respect to the total hemicellulose extracted.

The extracted hemicellulose was determined by subjecting the liquid fraction collected after enzymatic hydrolysis to a hydrolysis process with 4% $H_2SO_4$ at 121°C for 1 h. This acid hydrolysis treatment allows complex xylan polymers to be hydrolyzed to their most basic units: xylose and arabinose, mainly. The released monomers were separated and quantified by liquid chromatography (Agilent 1200 HPLC system, Waldbronn, Germany) using a Transgenomic ICSep ION-300 column (300 x 7.8 mm) (Chromtech, San Jose, CA, USA), as described in Rodriguez-Sanz, *A., Fuciños, C., Torrado, A. M.,* & *Rúa, M. L. (2022). **Extraction of the wheat straw hemicellulose fraction assisted by commercial endo-xylanases. Role of the accessory enzyme activities. Industrial Crops and Products, 179, 114655***. The total concentration of monosaccharides obtained allows quantifying the mg of hemicellulose extracted per gram of pretreated straw, following equation 2. The percentage of extracted hemicellulose with respect to the total composition of the pretreated material was calculated according to equation 3.

$$(2)\quad {mg\ Extracted\ hemicellulose}/{g\ pretreated\ solid} = ([X]_{AT} + [A]_{AT} + ([F]_{AT} \times 1.56)) \times \frac{V}{W}$$

$$(3)\quad \%\ Extracted\ hemicellulose = (Equation(2) \times \frac{1\ g\ pretreated\ solid}{292.9\ mg\ xylan}) \times 100$$

$[X]_{AT}$ is the concentration of quantified xylose after acid hydrolysis applied to the liquid obtained after the enzymatic treatment (expressed in mg / mL)

$[A]_{AT}$ is the concentration of quantified arabinose after acid hydrolysis applied to the liquid obtained after the enzymatic

treatment (expressed in mg / mL)
$[F]_{AT}$ is the concentration of furfural (expressed in mg / mL). This product is generated as a result of the acid hydrolysis treatment necessary for the quantification of the extracted hemicellulose
1.56 is the conversion factor that allows quantifying the xylose that has been transformed into furfural
V refers to the reaction volume (expressed in mL)
W refers to the weight of pre-treated raw material (expressed in g)
292.9 mg xylan per gram of solid is the total hemicellulose composition of the pretreated material used for enzymatic hydrolysis

Impurities (xylose and arabinose monomers) were quantified by directly analyzing the liquid fraction collected after enzymatic hydrolysis with the same chromatographic method described above. The mg of impurities per gram of pretreated straw ([Monom.]), and their percentage (% Monom.) with respect to the extracted hemicellulose were calculated according to equation 4 and 5, respectively.

$$(4)\quad [Monom.] = {mg\ Monomers}/{g\ pretreated\ solid} = ([M]_{EH}) \times \frac{V}{W}$$

$$(5)\quad \%\,Monom = \frac{Equation\ (4)}{Equation\ (2)} \times 100$$

$[M]_{EH}$ is the sum of concentrations of xylose or arabinose (expressed in mg / mL) present in the resulting liquid after enzymatic hydrolysis
V refers to the reaction volume (expressed in mL)
W refers to the weight of pre-treated raw material (expressed in g)

Finally, xylooligosaccharides (XOS) and arabinoxylooligosaccharides (AXOS) were analyzed directly after enzymatic hydrolysis with the Rezex RNO-Oligosaccharide Na+ (4%) column (200 x 10 mm) (Phenomenex, Alcobendas, Spain), with a refractive index detector. Specifically, the following were identified and quantified: (xylobiose ($X_2$), xylotriose ($X_3$), $3^2$ -α-L-arabinofuranosyl-xylobiose ($A^3X$), xylotetraose ($X_4$), $2^3$ -α-L-arabinofuranosyl-xylotriose ($A^2XX$), xylopentose ($X_5$), $2^3$,$3^3$-di-α-L-arabinofuranosyl-xylotriose ($A^{2,3}XX$) and xylohexose ($X_6$). Through this methodology, the mg of XOS-AXOS obtained per gram of pretreated solid and the percentage of XOS-AXOS obtained with respect to the total xylan extracted (equation 6 and 7, respectively) were quantified. The ADPs were determined by difference between the total extracted hemicellulose and the composition with respect to monomers and XOS-AXOS, following equation 8.

$$(6)\quad {mg\ (XOS-AXOS)}/{g\ pretreated\ solid} = (\sum([X_n]_{EH})) \times \frac{V}{W}$$

$$(7)\quad \%\,(XOS-AXOS) = \frac{\sum([X_n]_{EH} \times CF_n) \times \frac{V}{W}}{Equation\ (2)} \times 100$$

$$(8)\quad \%ADP = 100 - Equation\ (5) - Equation\ (7)$$

$[X_n]_{EH}$ is the concentration of each XOS or AXOS determined (in mg/mL) at the end of enzymatic hydrolysis and before acid treatment.
$CF_n$ is the conversion factor to xylose for each quantified XOS taking into account the release of water in the formation of the glycosidic bond: $CF_{X2}$= 1.06; $CF_{X3,\ A3X}$= 1.09; $CF_{X4,\ A2XX}$= 1.10; $CF_{X5\ and\ X6,\ A2,3XX}$ = 1.11
W refers to the pre-treated raw material weight (expressed in g) and
V refers to the reaction volume (expressed in mL).

The enzymatic hydrolysis treatment results are shown in Table 1.

Table 1. Results of different experiments of enzymatic hydrolysis of the pretreated wheat pay

| Exp | Hydrolysis conditions | | 2 hours | | | | 24 hours | | | |
|---|---|---|---|---|---|---|---|---|---|---|
| | T (°C) | Enzymatic Units[1] (U/g straw) | Rto extr. (%) | Resulting products (%) | | | Rto extr. (%) | Resulting products (%) | | |
| | | | | XOS | ADP | Imp.[2] | | XOS | ADP | Imp.[2] |
| PMC[3] | | | | | | | | | | |
| E1 | 40 | 200 | 25.0 | 97.1 | 0.0 | 2.9 | 47 | 93.2 | 0.0 | 6.8 |
| E2 | 40 | 2.000 | 36.4 | 93.5 | 0.0 | 6.5 | 54.6 | 87.9 | 0 | 12.1 |
| E3 | 65 | 200 | 16.8 | 74.7 | 23.9 | 1.4 | 17.6 | 52.9 | 45.5 | 1.6 |
| E4 | 65 | 400 | 21.6 | 62.5 | 35.9 | 1.6 | 27.5 | 54.9 | 43.6 | 1.5 |
| E7 | 65 (7h) + 40 | 200+200 | - | - | - | - | 53.8 | 70.1 | 25.1 | 4.8 |
| XynA3 [4] | | | | | | | | | | |
| E5 | 65 | 200 | 23.3 | 58.5 | 40.7 | 0.8 | 54.6 | 58.9 | 38.3 | 3.2 |
| E6 | 65 | 400 | 29.4 | 55.3 | 43.6 | 1.1 | 75.5 | 66.3 | 28.9 | 5.5 |

[1] Enzymatic units are quantified at process temperature.
[2] Xylose and arabinose monomers are considered impurities.
[3] PMC: *Pentopan® Mono concentrate.* Commercial thermostable mesophilic endo-xylanase
[4] XynA3. Thermophilic endo-xylanase.

Regarding the hydrolysis treatment results, it should also be noted that in none of the 7 examples considered in Table 1, concentrations were detected of the two carbohydrate degradation compounds (furfural and HMF) that are generated in thermal processes.

The use of pure endo-xylanases also prevents the generation of other monomers such as glucose from cellulose degradation. These results can be seen graphically in Figure 1, where a comparative graph of xylose and arabinose monomers generation ([Monom.]) from wheat straw hemicellulose (expressed in mg/g of pretreated material and in percentages with respect to the total hemicellulose extracted) is shown.

First, experiments E1 and E2 are shown, which reflect the described process in the temperature range 35-45°C, specifically in a preferred embodiment at 40°C. These processes employ a mesophilic enzyme, (*Pentopan® Mono Concentrate* - PMC), and reach a hemicellulose extraction yield of around 50% after 24 hours, with the resulting product being mainly xylooligosaccharides, with a composition above 85% of product, and the rest impurities consisting of a mixture of xylose and arabinose monomers.

This result is observed more clearly in experiment E2, where the number of enzyme units was multiplied by 10 (from 200 to 2000 U/g straw), slightly improving the extraction yield, but displacing the xylooligosaccharide production process to the xylose and arabinose monomers generation. In this experiment E2 it is seen how the amount of impurities in the product is increased, these impurities being understood as the xylose and arabinose monomers. In neither of these two experiments (E1 and E2) ADP generation is detected.

When the experiments at 40°C (experiments E1 and E2) are compared with those carried out in a single step in the range of 55-80°C, specifically at 65°C, and regardless of the enzyme (experiments E3 to E6) according to the present invention, ADP generation is observed, which leads to a decrease in % of generated XOS. Thus, the representation of XOS in the liquid resulting from enzymatic hydrolysis decreases from 87-94% to 52-66% with increasing temperature, regardless of the rest of conditions and extraction yields achieved.

In addition, it can be seen how, regardless of the other operating conditions, those processes according to the present invention, which comprise a hydrolysis step at a working temperature of 65°C, generate less monosaccharides, in percentage ratio to the total products generated (Figure 1). This translates into better quality final products in both the case of production of XOS-AXOS and ADPs, since the monomers are not considered prebiotics or have the technological properties.

When the effect of enzyme dose is compared, it is also observed that temperature is a determining factor in the improvement of hemicellulose extraction yield. Thus, while in experiments E1 and E2 corresponding to the operation at 40°C the 10-fold increase in the dose of enzyme (from 200 to 2,000 U) produces an improvement in extraction yield of 16%, doubling the dose of enzyme from 200 to 400 U at 65°C with the same thermostable mesophilic enzyme (experiments E3 and E4) causes an improvement in yield of 56%. Likewise, experiments E5 and E6, corresponding to working at 65°C with a thermophilic enzyme, increasing the enzyme dose by twice allows to improve the yield by 38%.

In any case, according to the experiments carried out, it is shown that the enzymatic treatment in the range described in the present invention, specifically exemplified at 65°C with the thermostable mesophilic xylanase, ADPs are generated, although with a low hemicellulose extraction yield. Therefore, in a preferred embodiment of the process for obtaining hemicellulose derivatives in one step according to the present invention a thermophilic xylanase is employed. This type of xylanase equals the yield obtained with the mesophilic xylanase at 40°C and 2,000U, with a 10-fold lower enzyme dose.

Although the increase in the number of enzyme units used in each case obtains the maximum possible extraction yield, this parameter is not the only factor in the process to be taken into account. The increase in the number of enzyme units means an increase in monomer generation, which are considered impurities for certain applications. Thus, in a preferred embodiment, the process according to the present invention is carried out with an enzymatic unit greater than 200 U/g of lignocellulosic material, preferably in the range 200-500 U/g pretreated solid, taking into account the total hydrolysis time.

In the same way, the hydrolysis time influences, where the more advanced the hydrolysis, the higher the extraction yield (with the exception of E3 and E4), but so is the production of monomers. Thus, in a preferred embodiment, regardless of the endo-xylanase employed, and taking into account the added enzymatic units, the time of the enzymatic hydrolysis step is comprised between 2 and 50 hours.

In the case of experiments with the thermostable mesophilic enzyme at 65°C (experiments E3 and E4), the time does not allow to improve the extraction yield due to the loss of enzymatic activity at that temperature after 2 h of hydrolysis. Therefore, in the present invention it is established as an alternative embodiment the use of thermostable mesophilic enzymes in the range of 55-65°C with successive enzymatic additions every 2-5 h that allow to correct the enzymatic inactivation and continue with the hydrolysis until results similar to those obtained with the thermophilic enzyme, in terms of extraction of the hemicellulose and composition of XOS-AXOS and ADPs.

The effect of consecutive addition can be seen in experiment E7, where in addition to adding two doses of the thermostable mesophilic enzyme PMC, the hydrolysis temperature has been varied. Thus, a first step is carried out at 65°C for 7 h and a second at 40°C for 17 h. As can be seen in Table 1, the use of two-step hydrolysis with a high and a low temperature achieves a significant increase in the extraction yield achieved with the mesophilic enzyme in just one step at 65°C (experiment E3), in addition to generating an intermediate product profile between the two defined situations (one-step experiments at 40°C -E1 to E2- and 65°C -E3 to E6-), generating both XOS and ADPs.

In this case, the additional incorporation of the second step manages to compensate for the limitation in the extraction of mesophilic enzymes at 65°C (Experiments E3 and E4) and raises the extraction yield above 50%, maintaining the modulation of generated products, obtaining a production of ADPs around 25%, a value much higher than those obtained in the current processes with a single step of 40°C.

Therefore, as reflected in these examples, when the hydrolysis is carried out at high temperature, regardless of the enzyme, dose or time used, a modulation of the composition shifted to the production of ADPs is obtained to the detriment of the generation of prebiotic XOS, highlighting again the advantage of working at high temperatures compared to the processes disclosed at present.

## Claims

1. Process for producing hemicellulose-derived products from a lignocellulosic material, **characterized in that** it comprises:

   a) an alkaline pretreatment of said lignocellulosic material to generate a hydrolysable solid, and
   b) an enzymatic hydrolysis of the solid resulting from step a) for hemicellulose extraction and production of hemicellulose-derived products;

   where the enzymatic hydrolysis of the resulting solid is carried out with an endo-xylanase at a temperature of 55-80°C.

2. Process for producing hemicellulose-derived products according to claim 1, wherein the enzymatic hydrolysis is carried out for a time of 2 - 50 hours.

3. Process for producing hemicellulose-derived products according to claim 1, wherein the endo-xylanase is a thermophilic enzyme.

4. Process for producing hemicellulose-derived products according to claim 1, wherein the endo-xylanase is a mesophilic enzyme.

5. Process for producing hemicellulose-derived products according to any of claims 1 to 4, where the step b) of enzymatic hydrolysis comprises an addition of 200-500 U per gram of solid resulting from step a).

6. Process for the directed production of hemicellulose-derived products according to any of claims 1 to 5, where the step of enzymatic hydrolysis b) comprises:

   B1) a first step of enzymatic hydrolysis in a temperature range of 55-80°C, and
   B2) a second step of enzymatic hydrolysis in a temperature range of 35-45°C.

7. Process for producing hemicellulose-derived products according to claim 6, wherein steps B1 and/or B2 are carried out with enzyme doses of 200-500 U/g solid pretreated for 2-50 h.

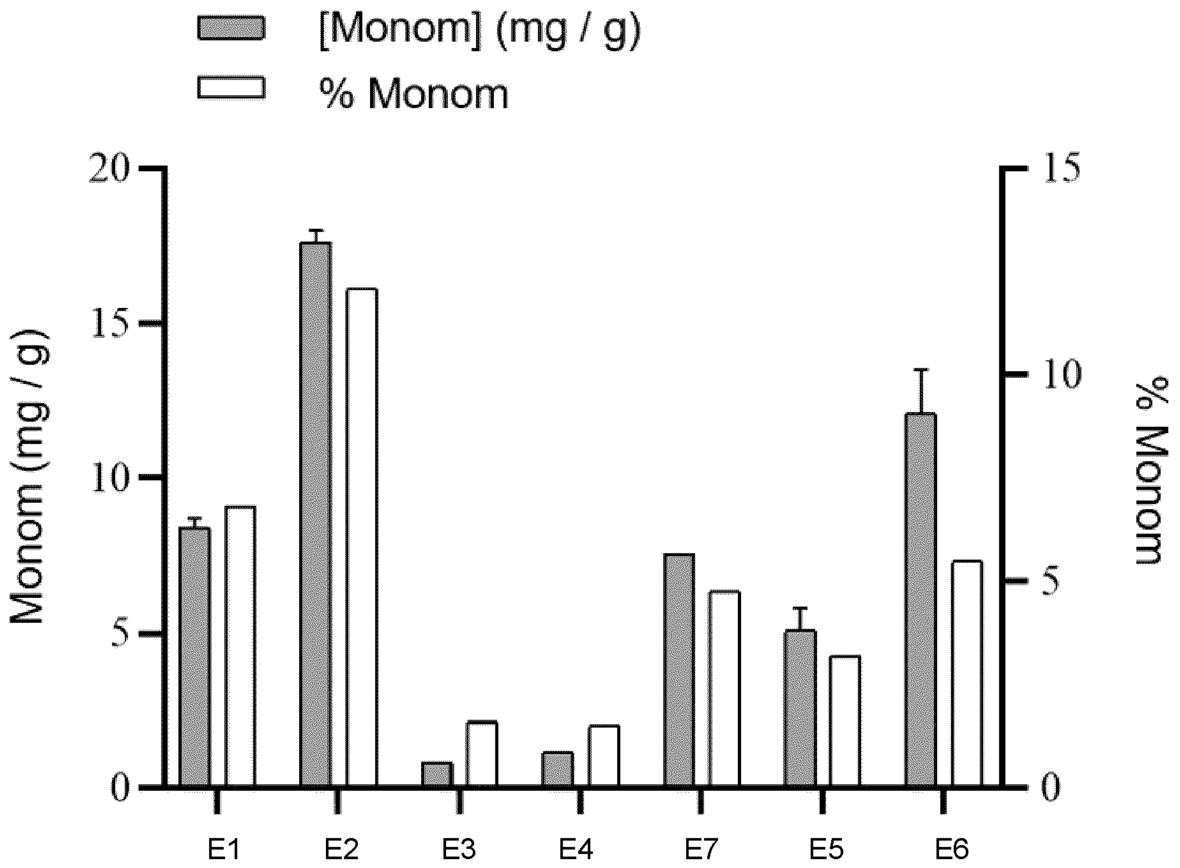
[Monom] (mg / g)
% Monom
Monom (mg / g)
20
15
10
5
0
% Monom
15
10
5
0
E1
E2
E3
E4
E7
E5
E6

Fig. 1

# INTERNATIONAL SEARCH REPORT

International application No.
PCT/ES2023/070562

A. CLASSIFICATION OF SUBJECT MATTER

See extra sheet

According to International Patent Classification (IPC) or to both national classification and IPC

B. FIELDS SEARCHED

Minimum documentation searched (classification system followed by classification symbols)
C12P, C12N, C07H, C08B, C08H

Documentation searched other than minimum documentation to the extent that such documents are included in the fields searched

Electronic data base consulted during the international search (name of data base and, where practicable, search terms used)

EPODOC, INVENES, WPI, CAPLUS

C. DOCUMENTS CONSIDERED TO BE RELEVANT

| Category* | Citation of document, with indication, where appropriate, of the relevant passages | Relevant to claim No. |
|---|---|---|
| X<br>Y | CN 108588144 A (GUANGZHOU INST ENERGY CONVERSION CAS) 28/09/2018, paragraphs [0004 - 0008]; examples 1-6. | 1-5<br>6, 7 |
| Y | WO 2010009515 A1 (APPLIMEX SYSTEMS PTY LTD et al.) 28/01/2010, pages 2 - 4; page 13; pages 15 - 16; page 22; page 25. | 6, 7 |
| A | CN 110016488 A (GUANGZHOU INST ENERGY CONVERSION CAS) 16/07/2019, paragraphs [0003 - 0009]; examples 1-6. | 1-7 |

☒ Further documents are listed in the continuation of Box C. ☒ See patent family annex.

* Special categories of cited documents:
"A" document defining the general state of the art which is not considered to be of particular relevance.
"E" earlier document but published on or after the international filing date
"L" document which may throw doubts on priority claim(s) or which is cited to establish the publication date of another citation or other special reason (as specified)
"O" document referring to an oral disclosure use, exhibition, or other means.
"P" document published prior to the international filing date but later than the priority date claimed
"D" document cited in the application.
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other documents , such combination being obvious to a person skilled in the art
"&" document member of the same patent family

| Date of the actual completion of the international search | Date of mailing of the international search report |
|---|---|
| 26/10/2023 | **(27/10/2023)** |
| Name and mailing address of the ISA/<br><br>OFICINA ESPAÑOLA DE PATENTES Y MARCAS<br>Paseo de la Castellana, 75 - 28071 Madrid (España)<br>Facsimile No.: 91 349 53 04 | Authorized officer<br>M. Taboada Rivas<br><br>Telephone No. 913495356 |

Form PCT/ISA/210 (second sheet) (July 2022)

**INTERNATIONAL SEARCH REPORT**

International application No.
PCT/ES2023/070562

C (continuation). DOCUMENTS CONSIDERED TO BE RELEVANT

| Category * | Citation of documents, with indication, where appropriate, of the relevant passages | Relevant to claim No. |
|---|---|---|
| A | REZA FARYAR et al... Production of prebiotic xylooligosaccharides fromalkaline extracted wheat straw using the K80R-variant of a termostable alkali-tolerantxylanase. Food And Bioproducts Processing, 22/11/2014, Vol. 93, pages 1-10, <DOI: http://dx.doi.org/10.1016/j.fbp.2014.11.004> See abstract; section 2.2; section 2.6. | 1-7 |
| D, A | RODRIGUEZ-SANZ ANDREA et al. Extraction of the wheat straw hemicellulose fraction assisted by commercial endo-xylanases. Role of the accessory enzyme activities. Industrial Crops And Products, 18/02/2022, Vol. 179, ISSN 0926-6690, <DOI:10.1016/j.indcrop.2022.114655> See abstract; section 2; section 3.4; table 3. | 1-7 |
| A | US 2012115192 A1 (LALI ET AL.) 10/05/2012, pages 4-10; example 3. | 1-5 |

**INTERNATIONAL SEARCH REPORT**

Information on patent family members

International application No.
PCT/ES2023/070562

| Patent document cited in the search report | Publication date | Patent family member(s) | Publication date |
|---|---|---|---|
| CN110016488 A | 16.07.2019 | CN110016488B B | 09.03.2021 |
| WO2010009515 A1 | 28.01.2010 | NONE | |
| CN108588144 A | 28.09.2018 | NONE | |
| US2012115192 A1 | 10.05.2012 | MY171749 A | 27.10.2019 |
| | | MY168527 A | 12.11.2018 |
| | | MY160407 A | 15.03.2017 |
| | | BRPI1014908 A2 | 31.01.2017 |
| | | ZA201109250 B | 29.08.2012 |
| | | US2013137147 A1 | 30.05.2013 |
| | | US8673596 B2 | 18.03.2014 |
| | | US2013078680 A1 | 28.03.2013 |
| | | US8709763 B2 | 29.04.2014 |
| | | JP2012527886 A | 12.11.2012 |
| | | KR20120036883 A | 18.04.2012 |
| | | KR101842080B B1 | 14.05.2018 |
| | | US8338139 B2 | 25.12.2012 |
| | | AU2010252547 A1 | 19.01.2012 |
| | | AU2010252547B B2 | 20.08.2015 |
| | | CA2763588 A1 | 02.12.2010 |
| | | CA2763588 C | 22.05.2018 |
| | | AR076925 A1 | 20.07.2011 |
| | | UY32669 A | 30.11.2010 |
| | | WO2010137039 A2 | 02.12.2010 |
| | | WO2010137039 A3 | 03.11.2011 |
| | | EP2438183 A2 | 11.04.2012 |

Form PCT/ISA/210 (patent family annex) (July 2022)

**INTERNATIONAL SEARCH REPORT**

International application No.

PCT/ES2023/070562

CLASSIFICATION OF SUBJECT MATTER

***C12P19/04*** (2006.01)
***C12P19/14*** (2006.01)
***C12N9/24*** (2006.01)
***C07H3/06*** (2006.01)
***C08B37/14*** (2006.01)
***C08H8/00*** (2010.01)

Form PCT/ISA/210 (extra sheet) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2015107413 A1
- ES 2364307 A1
- EP 3299394 A1
- CN 1680415 A
- WO 2007128025 A1

### Non-patent literature cited in the description


- **RIOS-RIOS, K. L.** ; **DEJONGHE, W.** ; **VANBROEKHOVEN, K.** ; **RAKOTOARIVONINA, H.** ; **RÉMOND, C.** Enzymatic Production of Xylo-oligosaccharides from Destarched Wheat Bran and the Impact of Their Degree of Polymerization and Substituents on Their Utilization as a Carbon Source by Probiotic Bacteria. *Journal of Agricultural and Food Chemistry*, 2021, vol. 69 (44), 13217-13226
- **ULBRICHT, R.J.** ; **NORTHUP, S.J.** ; **THOMAS, J.A.** A Review of 5-Hydroxymethylfurfural (HMF) in ParenteralSolutions. *Toxicol. Sci.*, 1984, vol. 4, 843-853
- **CHAPLA, D.** ; **PANDIT, P.** ; **SHAH, A.** Production of xylooligosaccharides from corncob xylan by fungal xylanase and their utilization by probiotics. *Bioresource Technology*, 2012, vol. 115, 215-221
- **YEGIN, S.** Microbial xylanases in xylooligosaccharide production from lignocellulosic feedstocks. *Biomass Conversion and Biorefinery*, 2022